# FASCICULE DE BREVET EUROPEEN

(11) **EP 0 839 041 B1**
(45) Date de publication et mention de la délivrance du brevet: **14.04.2004**
(21) Numéro de dépôt: 96925786.4
(22) Date de dépôt: 15.07.1996
(51) Int. Cl.: A61K 31/60, A61K 31/405, A61K 31/195, A61K 31/19, A61K 31/155

(54) **SELS DE DERIVES D'AMIDINE ET D'INHIBITEUR DE CYCLOOXYGENASE, LEUR PROCEDE DE PREPARATION, LEUR APPLICATION COMME MEDICAMENTS ET LES COMPOSITIONS PHARMACEUTIQUES LES RENFERMANT**
CYCLOOXYGENASEINHIBITOR UND SALZE VON AMIDINDERIVATEN, VERFAHREN ZU DEREN HERSTELLUNG, DEREN VERWENDUNG ALS ARZNEIWIRKSTOFFE SOWIE DIESE SALZE ENTHALTENDE ARZNEIMITTEL
CYCLO-OXYGENASE INHIBITOR AND AMIDINE DERIVATIVE SALTS, PREPARATION METHOD THEREFOR, USE THEREOF AS DRUGS, AND PHARMACEUTICAL COMPOSITIONS CONTAINING SAID SALTS

(30) Priorité: 15.07.1995 GB 9514518
(43) Date de publication de la demande: 06.05.1998
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHABRIER DE LASSAUNIERE, Pierre-Etienne, F-75016 Paris (FR); BROQUET, Colette, F-92100 Boulogne (FR)
(74) Mandataire: Bourgouin, André
(86) Numéro de dépôt international: PCT/FR1996/001095
(87) Numéro de publication internationale: WO 1997/003678

(56) Documents cités:
- GB-A- 2 263 111
- US-A- 4 324 801
- PATENT ABSTRACTS OF JAPAN vol. 010, no. 339 (C-385), 15 Novembre 1986 & JP,A,61 143320 (BANYU PHARMACEUT CO LTD), 1 Juillet 1986,
- PATENT ABSTRACTS OF JAPAN vol. 006, no. 024 (C-091), 12 Février 1982 & JP,A,56 147761 (BANYU PHARMACEUT CO LTD), 16 Novembre 1981,

## Description

La présente invention concerne de nouveaux composés présentant une double activité biologique, à savoir qu'ils inhibent à la fois la formation de monoxyde d'azote (NO) et l'activité des cyclooxygénases, un procédé pour leur préparation, des compositions pharmaceutiques les contenant et leur utilisation notamment en tant qu'inhibiteurs de NO synthase et de cyclooxygénase.

Les inhibiteurs de la cyclooxygénase ou des médicaments analogues de l'aspirine, c'est-à-dire l'acide acétylsalicylique et l'acide salicylique, les dérivés de l'indole méthylé, tels que l'indométacine (DCI de l'acide [1-(4-chlorobenzoyl)-5-méthoxy-2-méthyl-1H-indole-3-yl] acétique et le sulindac (DCI de l'acide [5-fluoro-2-méthyle-1-[[4-(méthylsulfinyle) phényle]-méthylène]-1H-indene-3-yl] acétique, les dérivés des acides N-phénylanthraniliques (méclofénamate, fénamates), les dérivés d'acide propionique tels que l'ibuprofène (DCI de l'acide p-isobutylhydratropique), naproxène, fénoprofène, sont largement employés et ont fait suffisamment leurs preuves comme médicament efficace en thérapie de l'inflammation, avec, cependant, quelques effets secondaires indésirables à des dosages élevés (R. Flower, S. Moncada et J. Vane. Mechanism of action of aspirin-like drugs - In the pharmacological basis of therapeutics Goodman and Gilman, 1985, 29, 674-715). En outre, ces composés ont été utilisés à la fois dans le traitement aigu et prophylactique de la migraine. La valeur de ces médicaments est indéniable, bien que leurs réponses thérapeutiques soient souvent incomplètes et que, chez certains malades, le traitement par de tels composés ne soit pas adapté. En raison de leurs propriétés anti-inflammatoire et anti-agrégante des plaquettes, ces composés ont également été utilisés pour la thrombose et avec une réduction évidente de l'oedème, dans les modèles d'ischémies du cerveau et, par conséquent, sont proposés dans le traitement et la prévention des infarctus, des commotions et des maladies cérébrovasculaires (W. Armstrong Recent trends in research and treatment of stroke, SCRIP, PJB publications. 1991).

L'activité biologique des inhibiteurs de la NO-synthase n'a été découverte que plus récemment et leur usage thérapeutique potentiel est seulement à l'étude. Ces substances, dont les structures sont représentées par des analogues de la L-arginine et décrites dans le brevet danois 3041/90, sont des inhibiteurs de la production d'oxyde nitrique (NO).

Notre connaissance actuelle sur le NO a été révisée en 1991, par Moncada et al, (S. Moncada, R.M.J. Palmer, E.A. Higgs : Nitric oxide : physiology, pathophysiology and pharmacology - Pharmacological reviews 43, 2, 109-142) et plus récemment par Kerwin et al. (Kerwin J., Lancaster J., Feldman P., Nitric oxyde : a new paradigm for second messengers, J. Med. Chem. (1995). vol. 38, 22. 4343-4362). En substance, il apparaît que la NO sert de mécanisme de transduction pour la guanylate cyclase soluble dans les plaquettes, le système nerveux, et de molécule effectrice dans les réactions immunologiques dans beaucoup de cellules et de tissus, y compris les macrophages et les neutrophiles. Le NO est produit enzymatiquement à partir de la L-arginine, par une enzyme appelée NO synthase. Cette enzyme existe sous deux formes : l'une constitutive (endothéliale et neuronale) et l'autre inductible. Dans certaines pathologies, une production excessive de NO peut se produire, comme cela a déjà été démontré au cours d'un choc. et tel que cela a été décrit dans le brevet précédemment cité. Dans ce contexte, les inhibiteurs de la NO synthase sont des médicaments efficaces pour prévenir des conséquences vasculaires et la mortalité causées par une maladie. en particulier lorsqu'ils sont combinés avec les inhibiteurs de la cyclooxygénase comme l'aspirine, l'indométacine ou le méclofénamate.

Les effets bénéfiques de la combinaison de deux principes actifs dans la même molécule. sont susceptibles de se produire pour les patients souffrant d'autres pathologies, comme par exemple :
- les troubles cardiovasculaires et cérébrovasculaires comprenant par exemple l'athérosclérose, la migraine, l'hypertension artérielle, le choc septique, les infarctus cardiaques ou cérébraux d'origine ischémique ou hémorragique, les ischémies et les thromboses ;
- les troubles du système nerveux central ou périphérique comme par exemple les maladies neurodégénératives où l'on peut notmment citer les infarctus cérébraux, les démences séniles, y compris la maladie d'Alzheimer, la chorée de Huntington, la maladie de Parkinson, la maladie de Creutzfeld Jacob, la sclérose latérale amyotrophique mais aussi la douleur, les traumatismes cérébraux ou de la moëlle épinière, l'addiction aux opiacées, à l'alcool et aux substances induisant une accoutumance, les troubles de l'érection et de la reproduction, les désordres cognitifs. les encéphalopathies ;
- les maladies prolifératives et inflammatoires comme par exemple l'athérosclérose, l'hypertension pulmonaire, la glomérulonéphrite, l'hypertension portale, le psoriasis, l'arthrose et l'arthrite rhumatoïde, les fibroses, les amyloïdoses, les inflammations du système gastrointestinal (colite, maladie de Crohn) ou du système pulmonaire et des voies aériennes (asthme. sinusites) ;
- les transplantations d'organe ;
- les maladies autoimmunes et virales comme par exemple le lupus. le sida, les infections parasitaires et virales, le diabète, la sclérose en plaque ;
- le cancer ; ou
- toutes les pathologies caractérisées par une production ou un dysfonctionnement de NO et/ou des cyclooxygénases.

Ainsi le brevet GB-A-2 263 111 décrit des sels de dérivés d'amidine et d'inhibiteur de cyclooxygénase, leur procédé de préparation ainsi que leur utilisation dans le traitement des troubles cardiovasculaires et cérébrovasculaires, des diverses formes d'inflammation ou des troubles du système immunitaire. Les inhibiteurs de cyclooxygénase décrits dans ce brevet sont : l'acide salicylique, l'acide acétylsalicylique, l'acide méfénamique, l'ibuprofène, l'indométacine et le sulindac; quant aux dérivés d'amidine, ils sont représentés par des analogues d'arginine (L-NO, L-NMMA, L-NAME).

L'invention a ainsi pour objet les produits de formule générale I

A.B (I)

sous forme de sel, dans laquelle
- A: représente un inhibiteur de cyclooxygénase présentant une fonction carboxy ;
- B: représente un composé de formule générale I_{B} 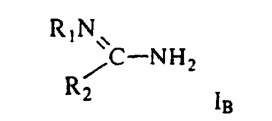 dans laquelle
R₁ représente H, le radical nitro ou phényle, le radical phényle étant éventuellement substitué par un ou plusieurs substituants choisis parmi les radicaux halo, cyano, nitro, trifluorométhyle, alkyle inférieur ou alkoxy inférieur ;
R₂ représente un radical amino substitué par un radical amino ou alkyl inférieur.

Dans les définitions indiquées ci-dessus. le terme halo représente le radical fluoro, chloro. bromo ou iodo, de préférence fluoro ou chloro.

L'expression alkyle inférieure représente de préférence un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle. Les radicaux alkoxy inférieurs peuvent correspondre aux radicaux alkyle indiqués ci-dessus. On préfère les radicaux méthoxy, éthoxy ou isopropyloxy.

L'invention a plus particulièrement pour objet les produits de formule générale I telle que définie ci-dessus, caractérisée en ce que le composé A est choisi parmi les salicylates tels que l'acide salicylique et ses dérivés, l'indométacine, le sulindac, les fénamates et les dérivés de l'acide propionique.

Parmi les dérivés de l'acide salicylique. on peut citer les composés obtenus par estérification de la fonction carboxy de l'acide salicylique comme, par exemple, le salicylate de méthyle, les composés obtenus par substitution du radical hydroxy de l'acide salicylique comme, par exemple, l'acide acétylsalicylique ou bien les composés obtenus par l'ajout de substituant(s) sur les sites libres du radical phényl de l'acide salicylique comme, par exemple, le diflunisale. Parmi les fénamates, on peut citer l'acide méfénamique, méclofénamique, flufénamique et tolfénamique. Comme exemples de dérivés de l'acide propionique, on peut citer les composés tels que ibuprofène, naproxène, fénoprofène, fenbufène, flurbiprofène, indoprofène, kétoprofène ou supropfène.

L'invention a particulièrement pour objet les composés de formule générale 1 telle que décrite ci-dessus. caractérisée en ce que
- A représente l'acide salicylique, le salicylate de méthyle, l'acide acétylsalicylique, l'indométacine, le sulindac, l'acide méfénamique, l'acide méclofénamique ou l'ibuprofène ; et
- B est de formule générale (I_{B}) telle que définie ci-dessus dans laquelle R₁ représente H, le radical nitro ou phényle, le radical phényle étant éventuellement substitué par un ou plusieurs substituants choisis parmi les radicaux chloro, tluoro, cyano, nitro, trifluorométhyle, méthyle, éthyle, méthoxy, éthoxy ou isopropyloxy ; et R₂ représente un radical hydrazino; méthylamino ou éthylamino.

Plus particulièrement. l'invention a pour objet les produits décrits ci-après dans les exemples, en particulier les produits répondant aux formules suivantes :
- salicylate d'aminoguanidine ;
- ibuprofénate d'aminoguanidine ;
- indométhacinate d'aminoguanidine ;
- acétylsalicylate de méthylguanidine ;
- salicylate de méthylguanidine ;
- ibuprofènate de méthylguanidine ;
- méfénamate de méthylguanidine.

L'invention a également pour objet un procédé de préparation des produits de formule générale I telle que définie ci-dessus, caractérisé en ce que l'on fait réagir dans l'eau, à une température comprise entre la température ambiante et 70 °C, un mélange du composé de formule A telle que définie ci-dessus avec un composé de formule B telle définie ci-dessus.

Lors de la mise en oeuvre du procédé, le composé de formule A peut être utilisé tel quel ou sous forme de sel comme, par exemple, le sel de sodium. De même, le composé de formule B peut être utilisé tel quel ou sous une forme comme, par exemple, son bicarbonate ou son hydrochlorure.

Les produits de formule A sont connus ou peuvent être fabriqués par les méthodes connues de l'homme de métier. Les produits de formule B peuvent être obtenus en appliquant les méthodes de préparation d'amidines connues de l'homme de métier (Schwan T.J. et coll.. J. Pharm. Sci. (1975). 64, 337-338 ; Roger R et coll., Chem. Rev. 61. 179, (1961) ; Tetrahedron, 29(14), 2147-51 (1973) ; Patai S., Chem. Amidines Imidates. vol.1, 283-348 (1975) ; Patai S., Chem. Amidines Imidates, vol.2, 339-366 (1990)).

Les composés de la présente invention possèdent d'intéressantes propriétés pharmacologiques. Ils présentent une double activité biologique, à savoir qu'ils inhibent à la fois le processus de la L-arginine / oxyde nitrique (NO) et le processus de la cyclooxygénase. Les composés de la présente invention peuvent ainsi être utilisés dans différentes applications thérapeutiques.

Compte tenu du rôle potentiel de la NO synthase et de la cyclooxygénase dans la physiopathologie, les composés selon l'invention peuvent produire des effets bénéfiques et favorables dans le traitement des :
- troubles cardiovasculaires et cérébrovasculaires. comprenant, par exemple, les migraines. les congestions cérébrales, les infarctus. les ischémies, les chocs septiques, endotoxiniques et hémorragiques, les douleurs ;
- diverses formes d'inflammation, comprenant, par exemple, les fièvres rhumatismales aiguës, les arthrites rhumatismales ou d'autres types d'arthrites, l'ostéo-arthrite, l'asthme;
- troubles du système immunitaire, comprenant les infections virales ou non virales, les maladies auto-immunes et toutes les pathologies caractérisées par une production excessive d'oxyde nitrique et/ou des métabolites d'acide arachidique.

On trouvera ci-après, dans la partie expérimentale, une illustration des propriétés pharmacologiques des composés de l'invention.

Ces propriétés rendent les produits de formule I aptes à une utilisation pharmaceutique. La présente demande a également pour objet, à titre de médicaments, les produits de formule I telle que définie ci-dessus ainsi que les compositions pharmaceutiques contenant, à titre de principe actif, l'un au moins des médicaments tels que définis ci-dessus.

L'invention concerne ainsi des compositions pharmaceutiques contenant un composé de l'invention. en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions. des suspensions ou des sirops. Les supports liquides appropriés peuvent être, par exemple. l'eau, les solvants organiques tels que le glycérol ou les glycols, de même que leurs mélanges, dans des proportions variées, dans l'eau, additionnés à des huiles ou des graisses pharmaceutiquement acceptables. Les compositions pharmaceutiques selon l'invention peuvent être administrées selon les modes d'administration classique telle administration orale ou par injection intramusculaire, intrapéritonéale, intraveineuse ou sous-cutanées.
L'invention a également pour objet l'utilisation des produits de formule I telle que définie ci-dessus, pour la préparation de médicaments destinés à traiter les troubles cardiovasculaires et cérébrovasculaires, de médicaments destinés à traiter diverses formes d'inflammation, de médicaments destinés à traiter les troubles du système immunitaire.

Les exemples suivants sont présentés pour illustrer les procédures ci-dessus.

### PARTIE EXPÉRIMENTALE :

### Exemple 1 : salicylate d'aminoguanidine

On dissout, en chauffant vers 70 °C, 420 mg (2,5 mmol) d'aminoguanidine bicarbonate dans 25 ml d'eau, puis on rajoute en agitant une solution aqueuse de 400 mg de salicylicate de sodium. On chauffe le mélange pendant 20 minutes puis on lyophilise (poudre blanche ; p.f. = 148° C).

### Exemple 2 : ibuprofénate d'aminoguanidine

On dissout, en chauffant vers 70 °C, 420 mg (2,5 mmol) d'aminoguanidine bicarbonate dans 25 ml d'eau. On rajoute une solution aqueuse de 570 mg de sel de sodium d'ibuprofène. On prolonge le chauffage pendant 20 minutes puis on lyophilise (poudre blanche ; p.f. = 134° C).

### Exemple 3 : indométhacinate d'aminoguanidine

On procède comme décrit dans l'exemple 1, mais en utilisant de l'indométhacine à la place du salicylicate de sodium (poudre jaune ; p.f. = 196° C).

### Exemple 4 : acétylsalicylate de méthylguanidine

On dissout 450 mg (2.5 mmol) d'acide acétylsalicylique dans 20 ml d'eau contenant 2.5 ml de NaOH 1 N. On rajoute, ensuite, 237,8 mg de chlorhydrate de méthylguanidine dans 10 ml d'eau. On poursuit l'agitation pendant 15 minutes. On obtient une solution limpide. La lyophilisation fournit une poudre blanche (p.f. = 153° C).
RMN-¹H (100 MHz. D₂O) : 7,3-6,6 (m, 4H, aromatique) ; 2,3 (s, 3H, NCH₃) ; 1,8 (s. 3H. COCH₃).

### Exemple 5 : salicylate de méthylguanidine

On dissout, à chaud. 345 mg (2.5 mmol) d'acide salicylique dans 20 ml d'eau contenant 2.5 ml de NaOH 1 N. On rajoute une solution de chlorhydrate de méthylguanidine (2,5 mmol) dans 10 ml d'eau. On poursuit l'agitation à 40 °C pendant 10 minutes. La lyophilisation fournit une poudre blanche très volumineuse (p.f. = 140° C).
RMN-¹H (100 MHz, D₂O) : 7-6.8 (m, 4H, aromatique) ; 2.4 (s, 3H, NCH₃).

### Exemple 6 : ibuprofènate de méthylguanidine

On dissout, à chaud. 2.5 mmol d'ibuprofen dans 20 ml d'eau contenant 2,5 ml de NaOH 1 N. On rajoute une solution de chlorhydrate de méthylguanidine (2.5 mmol) dans 10 ml d'eau. On chaffe en agitant à 60 °C pendant 15 minutes. La lyophilisation fournit une poudre blanche (p.f. = 174° C).
¹H-NMR (100 MHz. D₂O) : 7,2 (s, 4H, aromatique) ; 3,4 (q, 1H, C₆H₆-CH(Me)-CO) ; 2,6 (s, 3H. NCH₃) ; 2,25 (d, 2H. CH₂C₆H₆) ; 1,6 (m, 1H, CH(CH₃)₂) ; 1,2 (d, 3H, -CH(CH₃)-CO₂H) ; 0,8 (d, 6H. 2CH₃).

### Exemple 7 : méfénamate de méthylguanidine

On procède comme décrit dans l'exemple 6, mais en utilisant l'acide méfénamique à la place de l'ibuprofène. Au refroidissement. le composé précipite. On filtre et sèche le produit (p.f. = 124° C).
¹H-NMR (100 MHz, D₂O) : 8,1 (m, 1H, H en o de CO₂H) ; 7,2-6,8 (m, 6H, 2C₆H₆) ; 2,9 (s. 3H. NCH₃) ; 2,4 et 2,3 (d, 2CH₃C₆H₆).

En utilisant le procédé indiqué ci-dessus, on peut également préparer les produits suivants, qui font également partie de l'invention et qui constituent des produits préférés :

**Tableau 1**

| Composé | A | B B | |
|---|---|---|---|
| | | R₁ | R₂ |
| C | acide salicylique | nitro | hydrazino |
| D | acide salicylique | H | méthylamino |
| E | salicylate de méthyle | H | hydrazino |
| F | indométacine | 4-méthylphényle | hydrazino |
| G | indométacine | H | méthylamino |
| H | indométacine | nitro | éthylamino |
| I | sulindac | H | méthylamino |
| J | sulindac | H | hydrazino |
| K | acide méfénamique | nitro | hydrazino |

### Etude pharmacologique des produits de l'invention

Les composés de l'invention ont été soumis à des tests biologiques *in vitro*, afin de prouver leur activité à bloquer la NO synthase inductible et ta cyclooxygénase. Ils ont été comparés à des substances de références telles que l'aminoguanidine, la L-nitroarginine, l'ibuprofène, l'indométacine. l'acide acétylsalicylique.

### 1) Effet in vitro sur la NO synthase inductible de macrophages murins J774A1 :

Le test consiste à mesurer la transformation par la NO synthase de la L-arginine en L-citrulline d'après la méthode de Bredt et Snyder (Proceedings of the National Academy of Sciences USA 87, 682-685, 1990). Les macrophages murins J774A1 produisent une grande quantité de NO après activation par les lipopolysaccharides (LPS) et l'interféron-γ (IFN-γ). Les cellules sont cultivées dans du milieu DMEM (Dulbecco's Modified Eagle's Médium) enrichi avec 10 % de sérum de veau foetal à 37° C sous une atmosphère de 5 % de CO₂ après activation par le LPS et l'IFN-γ. Elles sont ensemencées à raison de 5000 cellules/cm² dans des flacons de 150 cm². Les incubations se font en présence de LPS (1 µg/ml) et d'IFN-γ murin (50 U/ml) dans du DMEM enrichi avec 10 % de sérum de veau foetal. La NO synthase est isolée avec un tampon d'extraction (HEPES 50 mM. pH 7,4, dithiothréital 0,5 mM. pepstatin A 1 mg/ml. leupeptine 1 mg/ml, inhibiteur de trypsine de soja 1 mg/ml. antipaïne 1 mg/ml et PMSF 10 mg/ml). Après sonication dans le tampon d'extraction à 4° C, les homogénats sont ultra-centrifugés ( 100 000 g à 4° C pendant 1 heure).

Le dosage se fait dans des tubes à essai en verre dans lesquels sont distribués 100 µl de tampon d'incubation contenant 100 mM d'HEPES, pH 7,4, 1 mM de dithiotréitol, 2.5 mM de CaCl₂, 10 µM de tétrahydrobioptérine. FAD 10 µM, BSA 1 mg/ml. 2 mM de NADPH réduit, 2 mM d'EDTA et 2,5 mM de CaCl₂. On ajoute 25 µl d'une solution contenant 100 nM d'arginine tritiée (activité spécifique : 56,4 Ci/mmole, Amersham) et 40 µM d'arginine non radioactive. La réaction est initiée en ajoutant 50 µl d'homogénat, le volume final étant de 200 µl (les 25 µl manquants sont soit de l'eau, soit le produit testé). Après 15 minutes, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide.

Les résultats sont exprimés en valeur de CI₅₀ et sont résumés dans le tableau du paragraphe 2 (première colonne de résultats intitulée "NO synthase inductible, formation de citrulline").

### 2) Effet in vitro sur la production de nitrites par les macrophages murins J774A1 :

Ce test est utilisé pour mesurer l'activité inhibitrice des produits sur la NO synthase inductible de cellules en cultures. Les cellules sont cultivées dans du milieu DMEM (Dulbecco's Modified Eagle's Medium) enrichi avec 10 % de sérum de veau foetal à 37° C sous une atmosphère de 5 % de CO₂. Pour les expériences. elles sont réparties en plaques 96 puits (50 000 cellules par puits) et incubées dans du DMEM sans rouge de phénol à 10 % de sérum de veau foetal avec du LPS (1 µg/ml) et de l'IFN-γ murin (50 U/ml) en présence ou en l'absence des produits à tester. Après 48 heures, la concentration de nitrites dans les milieux de culture, produits de dégradation du NO, est mesurée par une méthode colorimétrique selon Green et al, Analytical Biochemistry 126, 131-138 (1982). Les résultats sont exprimés en valeur de CI₅₀ et sont résumés dans le tableau du paragraphe 2 (deuxième colonne de résultats intitulée "NO synthase inductible, formation de nitrites").

| CI₅₀ (µM) | | |
|---|---|---|
| Produits | NO synthase inductible (formation de citrulline) | NO synthase inductible (formation de nitrites) |
| Aminoguanidine | 20 | 22 |
| L-nitroarginine | 21 | 100 |
| Ibuprofène | inactif | inactif |
| Indométacine | inactif | inactif |
| Exemple 2 | 11 | 16 |
| Exemple 3 | 26 | 33 |

### 3) Effet in vitro sur la production de nitrites et de PGE2 par des cellules microgliales de rat :

Les cellules microgliales sont isolées à partir de cultures de cellules gliales provenant de cortex de rat Wistar nouveau-né d'après le protocole de Théry et coll. (1991). Les cellules microgliales sont ensemencées dans des plaques 24 puits à raison de 5.10⁵ cellules par ml et 0,5 ml par puits. Les cellules microgliales sont incubées en présence de LPS (10 µg/ml) et des inhibiteurs pendant 24 heures à 37° C sous une atmosphère à 5 % de CO₂. Après 24 heures, les surnageants sont prélevés afin de mesurer les concentrations de nitrites et de PGE₂. La PGE₂ est dosée à l'aide d'un dosage radioimmunologique commercialisé par NEN selon le mode opératoire décrit par le fabricant. Chaque échantillon est dosé en duplicat. Les résultats sont exprimés sous forme de CI₅₀ calculées à l'aide d'une régression linéaire sur la partie linéaire de la courbe d'inhibition (logiciel FigP6C). Les nitrites sont dosés selon la méthode de Green et al (Analytical Biochemistry 126, 131-138, 1982). Les résultats sont exprimés sous forme de CI₅₀ et sont indiqués dans le tableau ci-dessous pour l'exemple 2.

| **Produit** | **Production de nitrites** **CI**_{**50**} **(µM)** | **Production de PGE**_{**2**} **CI**_{**50**} **(µM)** |
|---|---|---|
| Exemple 2 | 97 ± 35 | 3,1 ± 1,9 |
| Aminoguanidine | 109 ± 44 | inactif |
| Ibuprofène | inactif | 1,3 ± 0,22 |

### 4) Effet in vitro sur la cyclooxygénase inductible :

La cyclooxygénase existe sous deux isoformes, la COX-1 (constitutive) et la COX-2 induite par les agents inflammatoires mitogènes, cytokines et endotoxines. Les composés ont été testés sur l'activité enzymatique des deux isoformes semi-purifiées.

Le principe du test est de quantifier la transformation de l'acide arachidonique (AA) en PGE₂ par la COX-1 ou la COX-2. La méthode est inspirée de Futaki et al. (Prostaglandins, 47, 55-59, 1994). La COX-1 (Prostaglandine H synthase-1, EC 1.14.9.1) est conservée à -80° C et provient de vésicule séminale de bélier. La COX-2 (Prostaglandine H synthase-2) est aussi conservée à -80° C et provient de placenta de brebis.

Les tubes sont remplis avec 500 µl de tampon (100 mM de Tris HCl, pH 8, 1 µM d'hématine, 1 mM de phénol) et les composés de l'invention ou les substances de référence à des concentrations allant de 1 nM à 1 mM. Les témoins sont du tampon sans inhibiteurs. Après 2 minutes d'incubation avec 5 U (COX-2) ou 10 U (COX-1) d'enzyme, 5 µl d'acide arachidonique à 10 µM sont ajoutés pour 2 minutes. La réaction est arrêtée avec 30 µl d'HCl à 1N. L'extraction se fait sur des colonnes Seppack C18 (Waters). Après évaporation à sec, la PGE₂ est mesurée par dosage radioimmunologique à partir d'une trousse commerciale. Les résultats sont exprimés en valeur de CI₅₀ et sont résumés dans le tableau ci-dessous.

| **CI**_{**50**} **(µM)** | | |
|---|---|---|
| **Produits** | **COX-1** | **COX-2** |
| Acide acétylsalicylique | 77.8 | 648 |
| Acide salicylique | > 1000 | > 1000 |
| Acide méfénamique | 83.9 | 388 |
| Ibuprofène | > 1000 | > 1000 |
| Indométacine | 0.54 | 15.9 |
| Aminoguanidine | > 1000 | > 1000 |
| L-nitroarginine | > 1000 | > 1000 |
| Exemple 2 | 459 | 525 |
| Exemple 3 | 0.108 | 29.4 |
| Exemple 4 | 243 | > 1000 |
| exemple 5 | > 1000 | > 1000 |
| Exemple 6 | > 1000 | > 1 000 |
| Exemple 7 | 411 | 331 |

## Revendications

1. Les composés de formule générale I:
A.B (I)
sous forme de sel, dans laquelle
A représente un inhibiteur de cyclooxygénase présentant une fonction carboxy ;
B représente un composé de formule générale I_{B} 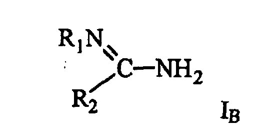 dans laquelle
R₁ représente H, le radical nitro ou phényle, le radical phényle étant éventuellement substitué par un ou plusieurs substituants choisis parmi les radicaux halo, cyano, nitro, trifluorométhyle, alkyle inférieur ou alkoxy inférieur ;
R₂ représente un radical amino substitué par un radical amino ou alkyl inférieur ,
étant entendu que le terme inférieur, lorsqu'il se réfère au radical alkyl ou alkoxy, signifie de 1 à 6 atomes de carbones.

2. Les composés de formule générale I telle que définie à la revendication 1, **caractérisée en ce que** le composé A est choisi parmi les salicylates, l'indométacine, le sulindac, les fénamates, ibuprofène, naproxène, fénoprofène, fenbufène, flurbiprofène, indoprofène, kétoprofène et supropfène.

3. Les composés de formule générale I telle que définie à l'une des revendications 1 ou 2, **caractérisée en ce que**
- A représente l'acide salicylique, le salicylate de méthyle, l'acide acétylsalicylique, l'indométacine, le sulindac, l'acide méfénamique, l'acide méclofénamique ou l'ibuprofène ; et
- B est de formule générale (I_{B}) telle que définie à la revendication 1 dans laquelle R₁ représente H, le radical nitro ou phényle, le radical phényle étant éventuellement substitué par un ou plusieurs substituants choisis parmi les radicaux chloro, fluoro, cyano, nitro, trifluorométhyle, méthyle, éthyle, méthoxy, éthoxy ou isopropyloxy ; et R₂ représente un radical hydrazino , méthylamino ou éthylamino ;

4. Les composés de formule générale I telle que définie à l'une des revendications 1 à 3 et répondant aux formules suivantes :
- salicylate d'aminoguanidine ;
- ibuprofénate d'aminoguanidine ;
- indométhacinate d'aminoguanidine ;
- acétylsalicylate de méthylguanidine ;
- salicylate de méthylguanidine ;
- ibuprofènate de méthylguanidine ;
- méfénamate de méthylguanidine.

5. Procédé de préparation des produits de formule générale I telle que définie à la revendication 1, **caractérisé en ce que** l'on fait réagir dans l'eau, à une température comprise entre la température ambiante et 70 °C, un mélange du composé de formule A telle que définie à la revendication 1 avec un composé de formule B telle définie à la revendication 1.

6. A titre de médicaments, les produits de formule I telle que définie à la revendication 1.

7. A titre de médicaments, les produits de formule I telle que définie à l'une des revendications 2 à 4.

8. Compositions pharmaceutiques contenant, à titre de principe actif, l'un au moins des médicaments tels que définis à l'une des revendications 6 ou 7.

9. Utilisation des produits de formule I telle que définie à l'une quelconque des revendications 1 à 4 pour la préparation de médicaments destinés à traiter les troubles cardiovasculaires et cérébrovasculaires, comprenant, par exemple, les migraines, les congestions cérébrales, les infarctus, les ischémies, les chocs septiques, endotoxiniques et hémorragiques, les douleurs.

10. Utilisation des produits de formule I telle que définie à l'une quelconque des revendications 1 à 4 pour la préparation de médicaments destinés à traiter les diverses formes d'inflammation comprenant les fièvres rhumatismales aiguës, les arthrites rhumatismales ou d'autres types d'arthrites, l'ostéo-arthrite, l'asthme.

11. Utilisation des produits de formule I telle que définie à l'une quelconque des revendications 1 à 4 pour la préparation de médicaments destinés à traiter les troubles du système immunitaire, comprenant les infections virales ou non virales, les maladies auto-immunes et toutes les pathologies **caractérisées par** une production excessive d'oxyde nitrique et/ou des métabolites d'acide arachidique.

## Patentansprüche

1. Die Zusammensetzungen der allgemeinen Formel I:
A.B (I)
in Form von Salz, in der
A einen Cyclooxygenase-Hemmer mit einer Carboxyfunktic darstellt;
B eine Verbindung der allgemeinen Formel I_{B} 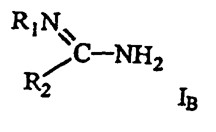 darstellt, in der
R₁ H, einen Nitrorest oder einen Phenylrest darstellt, wobei der Phenylrest ggf. durch einen oder mehrere Substituenten substituiert ist, die aus den Resten Halogen, Cyano, Nitro, Trifluormethyl, Niederalkyl oder Niederalkoxy ausgewählt sind;
R₂ einen durch einen Amino- oder Niederalkylrest substituierten Aminorest darstellt,
wobei gilt, daß der Begriff "nieder", wenn er sich auf den Alkyl- oder Alkoxyrest bezieht, 1 bis 6 Kohlenstoffatome bedeutet.

2. Die Verbindungen der allgemeinen Formel I, wie sie in Anspruch 1 definiert ist, **dadurch gekennzeichnet, daß** die Verbindung A aus den Salicylaten, Indometacin, Sulindac, den Fenamaten, Ibuprofen, Naproxen, Fenoprofen, Fenbufen, Flurbiprofen, Indoprofen, Ketoprofen und Suprofen ausgewählt ist.

3. Die Verbindungen der allgemeinen Formel I, wie sie in einem der Ansprüche 1 oder 2 definiert ist, **dadurch gekennzeichnet, daß**
- A Salicylsäure, Methylsalicylat, Acetylsalicylsäure, Indometacin, Sulindac, Mefenamsäure, Meclofenamsäure oder Ibuprofen darstellt; und
- B der in Anspruch 1 definierten allgemeinen Formel (I_{B}) entspricht, in der R₁ H, einen Nitrorest oder einen Phenylrest darstellt, wobei der Phenylrest ggf. durch einen oder mehrere Substituenten substituiert ist, die aus den Resten Chlor, Fluor, Cyano, Nitro, Trifluormethyl, Methyl, Ethyl, Methoxy, Ethoxy oder Isopropyloxy ausgewählt sind; und R₂ einen Hydrazino-, Methylamino- oder Ethylaminorest darstellt.

4. Die Verbindungen der allgemeinen Formel I, wie sie in einem der Ansprüche 1 bis 3 definiert ist, die den folgenden Formeln entsprechen:
- Aminoguanidinsalicylat;
- Aminoguanidinibuprofenat;
- Aminoguanidinindomethacinat;
- Methylguanidinacetylsalicylat;
- Methylguanidinsalicylat;
- Methylguanidinibuprofenat;
- Methylguanidinmefenamat.

5. Verfahren zur Herstellung der Produkte der allgemeinen Formel I, wie sie in Anspruch 1 definiert ist, **dadurch gekennzeichnet, daß** man eine Mischung der Verbindung der Formel A, wie sie in Anspruch 1 definiert ist, mit einer Verbindung der Formel B, wie sie in Anspruch 1 definiert ist, in Wasser bei einer Temperatur zwischen der Raumtemperatur und 70°C reagieren läßt.

6. Die Produkte der Formel I, wie sie in Anspruch 1 definiert ist, in Form von Medikamenten.

7. Die Produkte der Formel I, wie sie in einem der Ansprüche 2 bis 4 definiert ist, in Form von Medikamenten.

8. Pharmazeutische Zusammensetzungen, die als wirkstoff mindestens eines der Medikamente gemäß einem der Ansprüche 6 oder 7 enthalten.

9. Verwendung der Produkte der Formel I, wie sie in einem der Ansprüche 1 bis 4 definiert ist, für die Herstellung von Medikamenten, die für die Behandlung der kardiovaskulären und zerebrovaskulären Störungen bestimmt sind, die beispielsweise Migränen, zerebrale Kongestionen, Infarkte, Ischämien, endotoxische und hämorrhagische septische Schocks, Schmerzen umfassen.

10. Verwendung der Produkte der Formel I, wie sie in einem der Ansprüche 1 bis 4 definiert ist, für die Herstellung von Medikamenten, die für die Behandlung von verschiedenen Formen von Entzündungen bestimmt sind, die akute rheumatische Fieber, rheumatische Arthriten oder andere Arten von Arthritis, Osteoarthritis, Asthma umfassen.

11. Verwendung der Produkte der Formel I, wie sie in einem der Ansprüche 1 bis 4 definiert ist, für die Herstellung von Medikamenten, die für die Behandlung von Störungen des Immunsystems bestimmt sind, die die viralen oder nicht viralen Infektionen, Autoimmunerkrankungen und alle Krankheiten umfassen, die durch eine Überproduktion von Stickstoffmonoxid und/oder der Arachidinsäuremetaboliten **gekennzeichnet** sind.

## Claims

1. The compounds of general formula I:
A.B (I)
in the form of a salt, in which
A represents a cyclooxygenase inhibitor having a carboxy function;
B represents a compound of general formula I_{B} 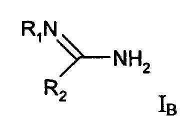 in which
R₁ represents H, the nitro or phenyl radical, the phenyl radical being optionally substituted by one or more substituents chosen from the halo, cyano, nitro, trifluoromethyl, lower alkyl or lower alkoxy radicals;
R₂ represents an amino radical substituted by amino or lower alkyl;
it being understood that the term "lower", when it refers to alkyl or alkoxy, means 1 to 6 carbon atoms.

2. The compounds of general formula I as defined in claim 1, **characterized in that** compound A is chosen from the salicylates, indomethacin, sulindac, the fenamates, ibuprofen, naproxen, fenoprofen, fenbufen, flurbiprofen, indoprofen, ketoprofen and supropfen.

3. The compounds of general formula I as defined in one of claims 1 or 2, **characterized in that**:
- A represents salicylic acid, methyl salicylate, acetylsalicylic acid, indomethacin, sulindac, mefenamic acid, meclofenamic acid or ibuprofen; and
- B is of general formula (I_{B}) as defined in claim 1 in which R₁ represents H, the nitro or phenyl radical, the phenyl radical being optionally substituted by one or more substituents chosen from the chloro, fluoro, cyano, nitro, trifluoromethyl, methyl, ethyl, methoxy, ethoxy or isopropyloxy radicals; and R₂ represents hydrazino, methylamino or ethylamino.

4. The compounds of general formula I as defined in one of claims 1 to 3 and corresponding to the following formulae:
- aminoguanidine salicylate;
- aminoguanidine ibuprofenate;
- aminoguanidine indomethacinate;
- methylguanidine acetylsalicylate;
- methylguanidine salicylate;
- methylguanidine ibuprofenate;
- methylguanidine mefenemate.

5. A process for the preparation of the products of general formula I as defined in claim 1, **characterized in that** a mixture of the compound of formula A as defined in claim 1 with a compound of formula B as defined in claim 1 is reacted in water at a temperature comprised between ambient temperature and 70°C.

6. As medicaments, the products of formula I as defined in claim 1.

7. As medicaments, the products of formula I as defined in one of claims 2 to 4.

8. Pharmaceutical compositions containing, as active ingredient, at least one of the medicaments as defined in one of claims 6 or 7.

9. Use of the products of formula I as defined in any one of claims 1 to 4 for the preparation of medicaments intended to treat cardiovascular and cerebrovascular disorders, including, for example, migraines, cerebral congestions, infarctions, ischemia, septic, endotoxinic and hemorrhagic shocks, pains.

10. Use of the products of formula I as defined in any one of claims 1 to 4 for the preparation of medicaments intended to treat various forms of inflammation, including, for example, acute rheumatoid fevers, rheumatoid arthritis or other types of arthritis, osteoarthritis, asthma.

11. Use of the products of formula I as defined in any one of claims 1 to 4 for the preparation of medicaments intended to treat disorders of the immune system, including viral or non-viral infections, auto-immune disorders and all the pathologies **characterized by** an excessive production of nitric oxide and/or metabolites of arachidic acid.
